(19) Europäisches Patentamt
European Patent Office
Office européen des brevets

(11) **EP 4 312 013 A1**

(12) **EUROPÄISCHE PATENTANMELDUNG**

(43) Veröffentlichungstag:
**31.01.2024   Patentblatt 2024/05**

(21) Anmeldenummer: 23186617.9

(22) Anmeldetag: **20.07.2023**

(51) Internationale Patentklassifikation (IPC):
*G01M 5/00* (2006.01)     *G01M 7/08* (2006.01)

(52) Gemeinsame Patentklassifikation (CPC):
**G01M 5/0066; G01M 5/0083; G01M 7/08**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **28.07.2022   DE 102022118916**

(71) Anmelder: **Hochschule Trier**
**54293 Trier (DE)**

(72) Erfinder:
• **LENZ, Philipp**
  **54329 Konz (DE)**
• **KOCH, Klaus-Peter**
  **66687 Wadern (DE)**
• **QUINTUS, Martin**
  **55469 Simmern (DE)**
• **MAIER, Christoph**
  **54298 Aach (DE)**

(74) Vertreter: **Durm Patentanwälte PartG mbB**
**Patentanwälte**
**Moltkestrasse 45**
**76133 Karlsruhe (DE)**

(54) **BESTIMMEN EINES ZUSTANDS EINES VERBUNDBAUTEILS**

(57)     Die vorliegende Erfindung betrifft eine Vorrichtung (10) zum Bestimmen eines Zustands eines Verbundbauteils (18), umfassend: eine Eingangsschnittstelle (12) zum Empfangen von Messdaten mit Informationen zu einem Zustand des Verbundbauteils (18); eine Analyseeinheit (14) zum Ermitteln eines Zustands des Verbundbauteils (18) basierend auf den Messdaten; und eine Ausgangsschnittstelle (16) zum Übermitteln des ermittelten Zustands; wobei die Messdaten eine elektrische Ladungsverschiebung und/oder elektrische Spannung umfassen, die durch eine mechanische Anregung (20) des Verbundbauteils (18) durch das Verbundbauteil erzeugbar ist; und die Analyseeinheit (14) dazu ausgebildet und eingerichtet ist, anhand der elektrischen Ladungstrennung und/oder elektrischen Spannung den Zustand des Verbundbauteils (18) zu bestimmen. Die vorliegende Erfindung betrifft ferner ein entsprechendes System sowie ein entsprechendes Verfahren.

Fig. 2

EP 4 312 013 A1

**Beschreibung**

Die vorliegende Erfindung betrifft eine Vorrichtung zum Bestimmen eines Zustands eines Verbundbauteils sowie ein entsprechendes System und Verfahren.

Eine Erfassung eines Zustands von Verbundmaterialien ist in vielen Anwendungen erforderlich. Insbesondere die innere Struktur, Änderungen der Einzelmaterialien und Materialverbindungen sind hierbei für die Festigkeit und mechanischen Eigenschaften der Verbundmaterialien von besonderem Interesse.

Hierzu ist es bekannt, anhand von Modellrechnungen und abgeschätzten Belastungshistorien einen Verschleiß der Verbundmaterialien abzuschätzen.

Ferner ist es bekannt, mit außen an den Verbundmaterialien angebrachten Sensoren und/oder von außen durchgeführten Messungen einen Verschleiß, eine Momentanbelastung oder Alterungseffekte zu bestimmen.

Die vorgenannten Möglichkeiten umfassen jedoch Näherungen und/oder punktuelle Messungen. Eine einfache und/oder direkte Bestimmung des Verbundmaterialzustandes, der Belastungshistorie und/oder der Momentanbelastung des Verbundmaterials ist bisweilen nicht ohne weiteres möglich.

Es besteht daher ein Bedarf, die Messung eines Zustands von Verbundmaterialien zu verbessern.

Die vorliegende Erfindung stellt sich daher die Aufgabe, eine Möglichkeit anzugeben, einen Zustand von Verbundmaterialien verbessert zu bestimmen. Insbesondere sollen dabei eine momentane mechanische Belastung, eine Belastungshistorie, Alterungen und/oder Störstellen erfassbar sein.

Die obige Aufgabe wird gelöst durch eine Vorrichtung zum Bestimmen eines Zustands eines Verbundbauteils, umfassend:

     eine Eingangsschnittstelle zum Empfangen von Messdaten mit Informationen zu einem Zustand des Verbundbauteils;

     eine optionale Eingangsschnittstelle zur Erfassung und/oder Erzeugung einer mechanischen Anregung des Verbundbauteils;

     eine Analyseeinheit zum Ermitteln eines Zustands des Verbundbauteils basierend auf den Messdaten und optional der mechanischen Anregung; und

     eine Ausgangsschnittstelle zum Übermitteln des ermittelten Zustands; wobei

     die Eingangsschnittstelle bevorzugt dazu geeignet sein kann, elektrische Ladungsverschiebungen und/oder elektrische Spannungen am Verbundmaterial zu erfassen und/oder Sensordaten eines Sensors zu empfangen;

     die mechanische Anregung durch die Vorrichtung oder den normalen Einsatz des Verbundbauteils erfolgen kann;

     die Messdaten eine elektrische Ladungsverschiebung und/oder elektrische Spannung umfassen, die durch eine mechanische Anregung des Verbundbauteils durch das Verbundbauteil erzeugbar ist; und

     die Analyseeinheit dazu ausgebildet und eingerichtet ist, anhand der elektrischen Ladungstrennung und/oder elektrischen Spannung den Zustand des Verbundbauteils zu bestimmen.

Weiterhin wird die obige Aufgabe gelöst durch ein System zum Bestimmen eines Zustands eines Verbundbauteils, mit:

     einer Vorrichtung wie zuvor definiert; und

     einer Anregeeinheit zum Einwirken auf das Verbundbauteil und Erzeugen einer vordefinierten mechanischen Anregung des Verbundbauteils.

Schließlich wird die obige Aufgabe gelöst durch ein Verfahren zum Bestimmen eines Zustands eines Verbundbauteils umfassend die Schritte:

     Empfangen von Messdaten mit Informationen zu einem Zustand des Verbundbauteils;

     Ermitteln eines Zustands des Verbundbauteils basierend auf den Messdaten; und

     Übermitteln des ermittelten Zustands; wobei

     die Messdaten eine elektrische Ladungstrennung und/oder elektrische Spannung umfassen, die durch eine mechanische Anregung des Verbundbauteils durch das Verbundbauteil erzeugt wird; und

     das Ermitteln eines Zustands des Verbundbauteils ein Bestimmen des Zustands des Verbundbauteils anhand der elektrischen Ladungstrennung und/oder elektrischen Spannung umfasst.

Insbesondere erfolgen eine Messung und ein Empfang der Anregung des Verbundbauteils.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere kann das Verfahren entsprechend der für die Vorrichtung und/oder das System in den abhängigen Ansprüchen beschriebenen Ausgestaltungen ausgeführt sein.

Es versteht sich, dass das Verfahren auch in Form eines Computerprogramms realisiert sein kann.

Durch eine Vorrichtung, die eine Eingangsschnittstelle, eine Analyseeinheit und eine Ausgangsschnittstelle umfasst, kann eine einfache und kostengünstige Vorrichtung geschaffen werden, die insbesondere in großer Stückzahl fertigbar ist und mobil einsetzbar ist. Dabei macht sich die Vorrichtung aktuelle Forschungsergebnisse, insbesondere der Hochschule Trier, zunutze. Es wurde festgestellt, dass mechanische Belastungen von leitenden und nichtleitenden Materialverbunden elektrische Ladungsverschiebungen erzeugen. Dabei steht die Menge der so entstandenen elektrischen Ladungstrennung bzw. elektrischen Ladungsverschiebung mit einer erfahrenen, insbesondere mechanischen, Belastung der Materialverbunde im Zusammenhang. Messtechnisch lassen sich diese elektrischen Ladungen oder elektrischen Spannungen erfassen und erlauben direkt Rückschlüsse auf den Zustand der Materialverbunde. Diese Erkenntnis wird in der Analyseeinheit genutzt, die dazu ausgebildet und eingerichtet ist, anhand der elektrischen Ladungstrennung und/oder elektrischen Spannung den Zustand des Verbundbauteils zu bestimmen. Hier können insbesondere Modelle, empirische Daten und/oder neuronale Netze genutzt werden, um die Daten zu analysieren. Es versteht sich, dass das Verbundbauteil vorzugsweise wenigstens ein elektrisch leitendes Material umfasst. Es versteht sich ferner, dass durch Anbringen eines leitenden Materials an einer Außenseite eines beliebigen Bauteils ein entsprechendes Verbundbauteil geschaffen werden kann. Der Zustand kann insbesondere wenigstens eine oder mehrere der folgenden Situationen umfassen: Das Verbundbauteil steht unter Krafteinwirkung bzw. ist mechanisch belastet, insbesondere durch eine akute dynamische Belastung. Das Verbundbauteil weist einen Verschleiß auf. Das Verbundbauteil weist Fehlstellen in einem Material des Verbundbauteils auf, das Verbundbauteil hat eine Belastungshistorie und/oder weist eine Alterung eines Materials des Verbundbauteils auf. Es versteht sich, dass weitere Situationen denkbar sind.

In einer vorteilhaften Ausgestaltung umfasst die Vorrichtung ein Messgerät, insbesondere in Form eines Ladungsverstärkers, eines Spannungsverstärkers und/oder eines Stromverstärkers, zum Erhöhen eines Messignals, insbesondere eine Spannungsamplitude der elektrischen Spannung. Durch ein Messgerät, insbesondere in Form eines Ladungsverstärkers, kann die elektrische Ladungstrennung bzw. eine anfallende elektrische Spannung verbessert und präziser erfasst werden. Insbesondere ermöglicht ein Messverstärker eine detailliertere Analyse einzelner Eigenschaften der elektrischen Ladungstrennung. Ferner kann, wenn eine größere Amplitude abgetastet wird, bei einer Erfassung der Spannungsamplitude eine höhere Auflösung des abgetasteten Signals erreicht werden. Eine Kombination der vorgenannten Messgeräte kann mittels einer definierten Abschlussimpedanz erfolgen.

Besonders bevorzugt ist die Eingangsschnittstelle zum Empfangen von Sensordaten mit Informationen zu einer mechanischen Anregung des Verbundbauteils ausgebildet, wobei die Analyseeinheit dazu ausgebildet und eingerichtet ist, basierend auf den Sensordaten den Zustand des Verbundbauteils zu bestimmen. Der Sensor kann insbesondere einen Sensor umfassen, der eine mechanische Anregung des Verbundbauteils bestimmt und entsprechende Sensordaten generiert. Hierdurch kann die entstandene elektrische Ladungstrennung und/oder elektrische Spannung in Relation zu der mechanischen Anregung gesetzt werden. Es kann eine detaillierte Analyse erfolgen. Insbesondere kann eine ohnehin schon auf das Verbundbauteil wirkende mechanische Anregung zur Bestimmung der Eigenschaften des Verbundbauteils genutzt werden.

Bevorzugt ist die Analyseeinheit dazu ausgebildet und eingerichtet, eine durch eine mechanische Anregung des Verbundbauteils erzeugte Mikrofonie zu quantifizieren und basierend auf der quantifizierten Mikrofonie einen Zustand vorzugsweise umfassend einen Verschleiß, Fehlstellen in einem Material, eine Belastungshistorie und/oder eine Alterung eines Materials des Verbundbauteils zu bestimmen. Hierdurch kann mit der Vorrichtung vorteilhaft ein Messstand geschaffen werden, bei dem keine zusätzlichen Sensoren benötigt werden, um einen Zustand eines Verbundbauteils, insbesondere direkt nach der Produktion, zu bestimmen.

Mit anderen Worten fungiert das Verbundbauteil selbst als Sensor und erzeugt bei mechanischer Anregung eine entsprechende elektrische Ladungstrennung, die Aufschluss über die vorgenannten Eigenschaften des Verbundbauteils erlaubt.

Es versteht sich, dass durch die Vorrichtung auch eine mobile Messstation geschaffen werden kann, die an ein Verbundbauteil angeschlossen wird. Sodann kann das Verbundbauteil mechanisch angeregt werden, beispielsweise durch einen Schlag oder durch sich wiederholende Schwingungen, insbesondere in Form von Vibrationen, Schallwellen oder dergleichen. Es kann folglich effektiv in kurzer Zeit ein Zustand eines Verbundbauteils, wie beispielsweise einer Brücke, ermittelt werden. Insbesondere ist es denkbar, eine Brücke auf Verschleiß in wiederkehrenden Abständen zu überprüfen. Hierdurch wird in vordefinierten Zeitabständen eine Vorrichtung wie zuvor beschrieben mit der Brücke leitend verbunden, wobei eine Anregung der Brücke durch Überfahren der Brücke mit einem Kraftfahrzeug erfolgt. In diesem Fall ist eine Messung ohne Absperrung der Brücke möglich.

Es versteht sich, dass auch eine vordefinierte Anregung erfolgen kann. Zudem kann die Anregung der Brücke durch einen weiteren Sensor erfasst werden, sodass die mechanische Anregung auf die Brücke quantifizierbar ist. Auch wenn vorliegend anhand des Beispiels einer Brücke die Funktionsweise der Vorrichtung erklärt wurde, ist dies keinesfalls einschränkend zu verstehen. Ein Fachmann erkennt, dass eine Vielzahl von Verbundmaterialien und/oder Verbundbauteilen mittels der Vorrichtung vermessbar sind.

In einer weiteren vorteilhaften Ausgestaltung ist die Analyseeinheit dazu ausgebildet und eingerichtet, eine durch eine vordefinierte mechanische Anregung des Verbundbauteils erzeugte Mikrofonie zu quantifizieren und basierend auf der quantifizierten Mikrofonie eine verbundbauteil-spezifische Kennlinie und/oder ein verbundbauteil-spezifisches Kennfeld zu bestimmen. Analog ist im Verfahren vorzugsweise der Schritt vorgesehen: Einwirken auf das Verbundbauteil mit einer vordefinierten Kraft, um eine vordefinierte mechanische Anregung des Verbundbauteils zu erzeugen; Quantifizieren einer durch die vordefinierte mechanische Anregung des Verbundbauteils erzeugten Mikrofonie; Bestimmen einer verbundbauteil-spezifischen Kennlinie und/oder eines verbundbauteil-spezifischen Kennfelds. Hierdurch können vorzugsweise unter Laborbedingungen Kennfelder für verschiedene Verbundmaterialien, wie beispielsweise Kabel, erstellt werden, wobei in Kenntnis dieser materialspezifischen Kennfelder und/oder Kennlinien auf einen Materialzustand eines bereits verlegten Kabels geschlossen werden kann. Die Vorrichtung befähigt einen Fachmann folglich, eine weitreichende Datenbank bezüglich verschiedener Verbundmaterialien anzulegen und anhand dieser Datenbank auf einen Verschleiß und/oder Zustand eines Verbundbauteils bei einer ersten Messung zu schließen. Hierzu ist vorzugsweise die Ausgangsschnittstelle dazu ausgebildet und eingerichtet, die verbundbauteil-spezifische Kennlinie und/oder das verbundbauteil-spezifische Kennfeld an einen Datenspeicher zu übermitteln. Analog ist vorzugsweise der folgende Schritt vorgesehen: Übermitteln der verbundbauteil-spezifischen Kennlinie und/oder des verbundbauteil-spezifischen Kennfelds an einen Datenspeicher. Es versteht sich, dass der Begriff "Datenspeicher" weit auszulegen ist. Ein Datenspeicher kann insbesondere eine Datenbank, einen Cloud-Speicher oder einen Wechselspeicher umfassen. Insbesondere ist es denkbar, dass zur Vermessung eines speziellen Kabeltyps ein Messgerät in Form einer Vorrichtung wie zuvor beschrieben geschaffen wird, wobei das verbundbauteil-spezifische Kennfeld und/oder die verbundbauteil-spezifische Kennlinie in einem Speicher der Vorrichtung hinterlegt ist.

Besonders bevorzugt ist ein Sensor zum Erfassen einer mechanischen Anregung des Verbundbauteils und zum Erzeugen von Sensordaten mit Informationen zu der mechanischen Anregung des Verbundbauteils vorgesehen, wobei die Vorrichtung dazu ausgebildet und eingerichtet ist, basierend auf den Sensordaten den Zustand des Verbundbauteils zu bestimmen. Hierdurch kann ein System geschaffen werden, das vorzugsweise autark bei jedweder mechanischen Anregung des Verbundbauteils einen Zustand des Verbundbauteils bestimmt. Mit anderen Worten kann ein Überwachungssystem geschaffen werden, das bei jedweder mechanischen Anregung des Verbundbauteils einen Zustand des Verbundbauteils ermittelt. Es versteht sich, dass ein derartiges System insbesondere dazu ausgebildet sein kann, einen Verschleiß des Verbundbauteils zu bestimmen und eine entsprechende Meldung an eine Wartungseinheit zu übermitteln, wenn der ermittelte Verschleiß des Verbundbauteils nahe an einem vordefinierten zulässigen Grenzwert für einen Verschleiß des Verbundbauteils ist.

Vorteilhafterweise umfasst ein Verfahren den Schritt: Empfangen von Sensordaten mit Informationen zu einer mechanischen Anregung des Verbundbauteils, wobei das Ermitteln eines Zustands des Verbundbauteils ein Bestimmen des Zustands des Verbundbauteils basierend auf den Sensordaten umfasst. Durch das Empfangen von Sensordaten kann eine mechanische Anregung des Verbundbauteils präzise bestimmt werden. Das Ermitteln eines Zustands des Verbundbauteils basierend auf den Sensordaten ermöglicht eine hochpräzise Analyse des Momentanzustands des Verbundbauteils.

Besonders bevorzugt umfasst der Schritt Ermitteln eines Zustands des Verbundbauteils ein Bestimmen einer einwirkenden mechanischen Belastung, vorzugsweise basierend auf einer verbundbauteil-spezifischen Kennlinie und/oder eines verbundbauteil-spezifischen Kennfelds. Hierdurch kann eine mechanische Belastung anhand des Kennfelds und/oder der Kennlinie quantifiziert werden. Es versteht sich, dass auch eine Quantifizierung ohne Kennlinie oder Kennfeld möglich ist. Insbesondere bei Brücken kann hier beispielsweise eine Verkehrsüberwachung erfolgen, da eine mechanische Belastung beim Überfahren durch einen LKW sich stark von der mechanischen Belastung beim Überfahren mit einem Fahrrad unterscheidet. Durch die Berücksichtigung einer Kennlinie und/oder eines Kennfelds kann die mechanische Anregung präzisiert werden.

Vorteilhafterweise umfasst der Schritt Ermitteln eines Zustands des Verbundbauteils ein Bestimmen eines Verschleißes, einer Fehlstelle in einem Material, einer Belastungshistorie und/oder einer Alterung eines Materials des Verbundbauteils, basierend auf einer beliebigen mechanischen Anregung, vorzugsweise basierend auf einer verbundbauteil-spezifischen Kennlinie und/oder eines verbundbauteil-spezifischen Kennfelds. Hierdurch kann das Verbundbauteil im Betrieb auf die vorgenannten Zustände überwacht werden, insbesondere kann ein Verschleiß des Verbundbauteils im Betrieb überwacht werden. Durch eine bauteil-spezifische Kennlinie oder ein bauteil-spezifisches Kennfeld kann der Verschleiß des Verbundbauteils hochpräzise ermittelt werden. Insbesonsdere kann hierdurch erreicht werden, dass Verbundbauteile erst dann ausgetauscht werden, wenn sie an der Verschleißgrenze sind. Ein präventives Austauschen von Verbundbauteilen kann vorzugsweise entfallen.

Mit der vorgestellten Lehre können der Zustand von Material und Verbundzustände von Materialverbänden über die Erfassung von mechano-elektrischen Signalen bzw. der Mikrofonie bestimmt werden. Der neu entwickelte Messaufbau kann an bereits bestehenden Anlagen nachgerüstet werden und mit den zu untersuchenden Materialverbänden verklemmt werden. Es versteht sich, dass die Messung ständig als Qualitätskontrolle nach der Fertigung oder im Rahmen von vorbeugenden Instandhaltungsmaßnahmen mit vordefinierten zeitlichen Abständen erfolgen kann. Unter Mikrofonie sind insbsondere alle mechano-elektrischen Effekte zu verstehen.

Der fertigungs-, belastungs- sowie altersbedingte Ausfall komplexer Strukturen bzw. Verbundmaterialien kann durch diesen Messaufbau verhindert werden und eine Funktionseinschränkung rechtzeitig erkannt werden.

Vorzugsweise sollen durch das neu entwickelte Sensor- bzw. Messprinzip in gefährdeten oder relevanten mechanischen Strukturen, Bauteilen und sich in Bewegung befindlichen Komponenten die dynamischen Abnutzungen oder Fehlstellen erfasst werden. Es versteht sich, dass bei kritischen Abnutzungsgraden oder Fehlstellen eine mit der Vorrichtung verbundene Hardware oder die Vorrichtung selbst eine Warnung ausgeben und den Verschleißgrad bzw. eine restliche Nutzungsdauer angeben kann.

Insoweit ist unter dem Begriff "Ermitteln eines Zustands" das Ermitteln einer restlichen Nutzungsdauer zu verstehen.

Insbesondere liegt der vorliegenden Lehre die Erkenntnis zugrunde, dass mechanische Belastungen von leitenden und nichtleitenden Materialverbünden elektrische Ladungsverschiebungen erzeugen. Die Menge, der zeitliche Verlauf bzw. die Ausprägung der so entstehenden elektrischen Ladungstrennung steht im Zusammenhang zur erfahrenen Belastung der Verbundmaterialien. Diese elektrischen Ladungen und/oder elektrischen Spannungen lassen sich zuverlässig messtechnisch erfassen und erlauben direkte Rückschlüsse auf den Zustand der Materialverbünde.

Es versteht sich, dass weitere Effekte, wie eine Temperatur, insbesondere eine Bauteiltemperatur, bevorzugt bedingt durch eine Umgebungstemperatur und/oder Sonnenstrahlung; eine Luftströmung, insbesondere eine Oberflächenwindgeschwindigkeit einer Luftbewegung an Windrädern; und/oder Feuchte, insbesondere eine Luftfeuchte und damit auch indirekt eine Bauteilfeuchte, einen Einfluss auf die Messdaten haben können. Folglich können die vorgenannten Zustände basierend auf ihrem Einfluss auf die Messdaten und dem Zusammenhang des Zustands des Verbundmaterials mit der erzeugten elektrischen Ladungstrennung und/oder elektrischen Spannung ermittelt werden und/oder der Einfluss auf die Messdaten bei der Analyse der Messdaten berücksichtigt werden.

Ferner versteht sich, dass die weiteren Effekte, wie eine Temperatur, eine Luftströmung und/oder eine Feuchte, durch externe Sensoren ermittelbar sind.

Vorzugsweise erlaubt eine Korrelation der einwirkenden mechanischen Belastung mit der gemessenen elektrischen Ladung eine spezifische Analyse der entstehenden Mikrofonie und ihrer Quantifizierung. In einem hierfür entwickelten Messstand können folglich Kennlinien und/oder Kennfelder für Kabel und ähnliche Materialverbünde angefertigt und als Referenz für den Anwendungsfall in die Auswerteelektronik eingebracht werden.

Eine Einkopplung externer Belastungen, also einer mechanischen Anregung, kann über elektro-, magneto-, opto- oder thermo-mechanische Effekte oder direkt mechanisch erfolgen. Es versteht sich, dass hierbei auch autonome Anwendungen möglich sind, bei denen bereits in einem System vorhandene Effekte als Signalquellen verwendet und korreliert werden.

Das ausgewertete Signal erlaubt eine Aussage über die dem Sensor, also dem Verbundmaterial, zugefügte akkumulierte Belastungshistorie. Es kann also sowohl der momentane Belastungszustand als auch eine in der Vergangenheit liegende Belastungshistorie bestimmt werden.

Mit der vorliegenden Lehre werden mechano-elektrische Effekte in oder an einem zu prüfenden Prüfobjekt genutzt, um einen Zustand des Prüfobjekts zu beurteilen. Hierbei können Veränderungen in einem oder in beiden Materialien des Verbundmaterials erkannt werden, die direkt oder indirekt eine Auswirkung auf die mechano-elektrischen Effekte haben.

Besonders bevorzugt kann eine Überprüfung von Fehlstellen oder Veränderungen in Verbundmaterialien in einer neuen Prüfklasse geschaffen werden.

Quantifizierung ist insbesondere ein Angeben von Eigenschaften, Beschaffenheiten und/oder Ausprägungen als Zahlenwert. Vom Begriff Quantifizierung kann auch eine Quantisierung, also Digitalisierung einer analogen Messgröße, umfasst sein.

Die hier offenbarte Lehre bietet insbesondere wenigstens einen der folgenden Vorteile. Es kann ein rechtzeitiges, nicht präventives, Austauschen von Bauteilen, Strukturen, Medienschläuchen etc. erfolgen. Es ist eine Erfassung von mechanischen Verschleißmechanismen möglich. Die Vorrichtung kann einfach installiert werden. Vorzugsweise kann eine ständige Überwachung durch Verwendung bereits auftretender Bewegungen als Referenz erfolgen. Ein Erfassen von Fehlstellen direkt nach der Produktion ist möglich. Es kann ein Erkennen von Lastspitzen erfolgen, die insbesondere zur inneren Schädigung eines Bauteils führen können. Hierdurch können Folgeschäden und Anlagenausfälle vermieden werden.

Ein Fachmann erkennt, dass die offenbarte Lehre in einer Vielzahl von Anwendungsgebieten vorteilhaft eingesetzt werden kann, wie unter anderem bei Windrädern, bei Gebäuden, bei Brücken, und/oder im Fahrzeugbau. Also mithin sowohl bei statischen als auch dynamischen Systemen.

Gemäß der offenbarten Lehre wird ein Verbundbauteil als Sensor benutzt, um seinen Zustand, also den Zustand des Verbundbauteils, selbst zu erfassen. Hierbei wird eine Ladungsverschiebung und/oder elektrische Spannung im Verbundbauteil, insbesondere bei einem Übergang zwischen zwei Materialien oder mehreren Materialien des Verbundbauteils, gemessen. Es versteht sich, dass auch ein Leiter in das Verbundmaterial eingracht werden kann. Veränderungen im Verbundbauteil oder einem Verbundwerkstoff können Auswirkungen auf mechano-elektrische Effekte haben. Diese Auswirkungen können zu einer Veränderung der Ladungsverschiebung und/oder elektrischen Spannung führen. Hierdurch kann, basierend auf einer Änderung der Ladungsverschiebung und/oder elektrischen Spannung, auf einen mechano-elektrischen Effekt und dadurch auf eine Veränderung im Verbundbauteil oder Verbundwerkstoff geschlossen werden. Es können insbesondere Störstellen, Fehlstellen, Defekte, eine Alterung eines oder mehrerer Materalen des Verbundbauteils und/oder eine Belastungshistorie erkannt werden.

Im Folgenden wird ein Ausführungsbeispiel der Erfindung unter Bezugnahme auf die beigefügten Abbildungen beschrieben. Es zeigen:

Figur 1     eine schematisch vereinfachte Darstellung einer Vorrichtung zum Bestimmen eines Zustands eines Verbundbauteils;

Figur 2     eine schematisch vereinfachte Darstellung einer Vorrichtung und eines Verbundbauteils;

Figur 3     eine schematisch vereinfachte Darstellung eines Systems zum Bestimmen eines Zustands eines Verbundbauteils;

Figur 4     schematisch ein Flussdiagramm zur Bestimmung eines Zustands eines Verbundbauteils;

Figur 5     schematisch ein weiteres Flussdiagramm zur Bestimmung eines Zustands eines Verbundbauteils;

Figur 6     schematisch die Schritte eines erfindungsgemäßen Verfahrens; und

Fig. 7a-7e     schematische Schaubilder eines Biegeversuchs.

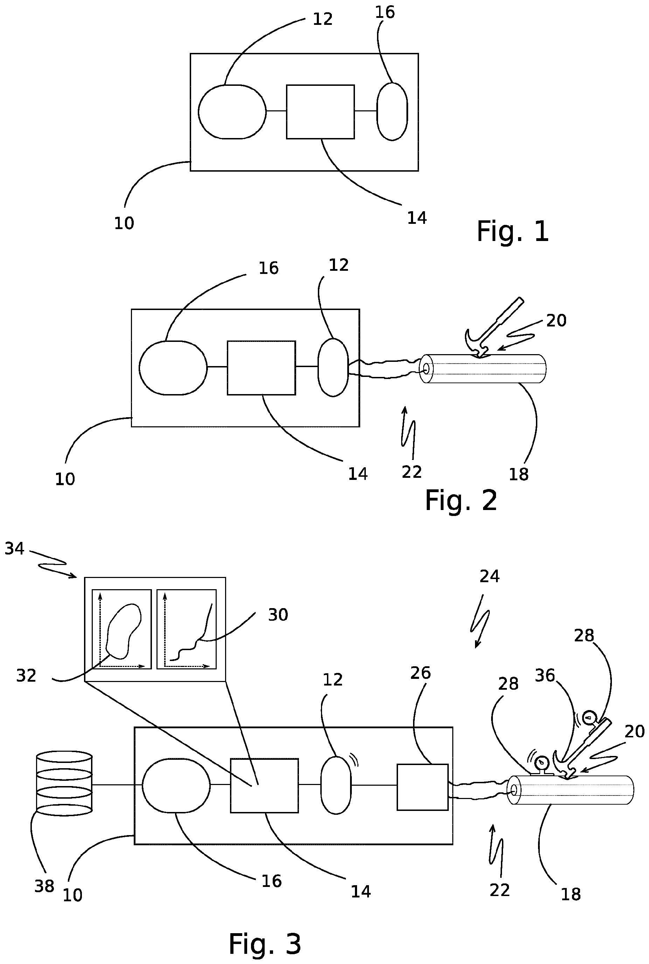

In Figur 1 ist eine Vorrichtung 10 zum Bestimmen eines Zustands eines Verbundbauteils schematisch vereinfacht dargestellt.

Die Vorrichtung 10 umfasst eine Eingangsschnittstelle 12, eine Analyseeinheit 14 und eine Ausgangsschnittstelle 16.

Die Eingangsschnittstelle 12 ist dazu ausgebildet, Messdaten mit Informationen zu einem Zustand des Verbundbauteils zu empfangen. Die Messdaten umfassen insbesondere eine elektrische Ladungsverschiebung, eine elektrische Ladungstrennung und/oder eine elektrische Spannung, die durch eine mechanische Anregung des Verbundbauteils erzeugt wird.

Die Analyseeinheit 14 ermittelt anhand der elektrischen Ladungsverschiebung, elektrischen Ladungstrennung und/oder elektrischen Spannung den Zustand des Verbundbauteils. Hierzu bedient sich die Analyseeinheit verschiedener bekannter Analysemethoden. Es versteht sich, dass die Analyseeinheit dazu ausgebildet und eingerichtet ist, beispielsweise eine Höhe und/oder Abklingzeit, einen Verlauf der elektrischen Ladungstrennung über die Zeit, eine Pulsweite, ein Spektrum, einen Effektivwert oder dergleichen zu verarbeiten, beispielsweise mittels lernender Algorithmen bzw. maschinellem Lernen, und anhand der ermittelten Parameter den Zustand, also insbesondere die Höhe der Krafteinwirkung, einen Verschleiß, Fehlstellen in einem Material und/oder eine Belastungshistorie oder Alterungen eines Materials des Verbundbauteils zu bestimmen.

In Figur 2 ist eine Vorrichtung 10 mit einem Verbundbauteil 18 dargestellt. In dem gezeigten Beispiel umfasst das Verbundbauteil 18 ein Kabel mit einem Schirm und einem Kern. Aus Gründen der Übersicht ist ein Mantel des Kabels nicht dargestellt. Es versteht sich, dass insbesondere die Interaktion zwischen Leiter und Isolator, z. B. Mantel, für den Triboelektrischen Effekt und nur der Isolator für den Piezoelektrischen Effekt maßgebend sein können und die gewählte Darstellung der besseren Übersicht dient.

Schematisch dargestellt ist eine mechanische Anregung 20 in Form einer Krafteinwirkung auf das Verbundbauteil 18. Zum Erfassen einer elektrischen Ladungsverschiebung und/oder elektrischen Spannung ist die Eingangsschnittstelle 12 über zwei Kabel 22 mit der Schirmung und dem Kern des Verbundbauteils 18 verbunden. Es versteht sich, dass auch nur ein Kabel vorgesehen sein kann, um die elektrische Ladungsverschiebung und/oder elektrische Spannung zu erfassen. Es versteht sich weiter, dass das Verbundbauteil 18 auch nur ein Material umfassen kann und ein Kabel beispielsweise an der Außenseite des Verbundbauteils 18 angebracht wird, um aus dem Single-Material-Bauteil, also einem Bauteil, das aus nur einem Material gefertigt ist, ein Verbundbauteil 18 zu schaffen. Alternativ kann ein Leiter im Verbundmaterial integriert sein und ein weiterer Leiter außen angebracht werden.

Aufgrund der schematisch dargestellten Krafteinwirkung wird im Verbundbauteil 18 eine elektrische Ladungsverschiebung und/oder elektrische Spannung, insbesondere eine Mikrofonie, erzeugt, die durch die Eingangsschnittstelle 12 zur Analyseeinheit 14 übermittelt werden kann. Die Analyseeinheit 14 ermittelt dann, wie zuvor beschrieben, einen Zustand des Verbundbauteils 18.

In Figur 3 ist schematisch ein System 24 zum Bestimmen eines Zustands des Verbundbauteils 18 dargestellt.

In der gezeigten Ausführungsform umfasst die Vorrichtung 10 eine Messeinheit, insbesondere einen Ladungsverstärker 26.

Der Ladungsverstärker 26 ist dazu ausgebildet, eine elektrische Ladungsverschiebung und/oder elektrische Spannung zu erhöhen, insbesondere eine Amplitude zu erhöhen, sodass die Analyseeinheit 14 eine detailliertere Analyse durchführen kann. Insbesondere können hierbei Effekte durch das Erfassen der elektrischen Ladungsverschiebung und/oder elektrischen Spannung verringert werden und ein Signal-zu-Rausch-Verhältnis des gewünschten Signals erhöht werden.

Das System 24 umfasst ferner optionale Sensoren 28, die die mechanische Anregung 20 erfassen können. Insbesondere können die Sensoren 28 eine Stärke der mechanischen Anregung bestimmen. Hierfür kommunizieren die Sensoren 28, in dem gezeigten Beispiel kabellos, mit der Eingangsschnittstelle 12. Es versteht sich, dass auch nur einer der beiden optionalen Sensoren 28 Anwendung finden kann.

Durch die bekannte Ausprägung der mechanischen Anregung 20, die beispielsweise beim Betrieb des Verbundbauteils 18 stattfindet, kann die Analyseeinheit 14 eine Kennlinie 30 und/oder ein Kennfeld 32 für das Verbundbauteil 18 bestimmen. Schematisch sind die Kennlinie 30 und das Kennfeld 32 in einem Schaubild 34 dargestellt.

Zur Bestimmung der Kennlinie 30 und/oder des Kennfelds 32 kann auch eine Anregeeinheit 36 Anwendung finden, die dazu ausgebildet ist, eine vordefinierte mechanische Anregung 20 im Verbundbauteil 18 zu erzeugen. Hierbei kann die vordefinierte mechanische Anregung 20 eine einzelne punktuelle Anregung, eine flächige Anregung oder eine zeitlich variierende Anregung, insbesondere ein Vibrieren oder dgl., umfassen.

Die Ausgangsschnittstelle 16 ist mit einem optionalen Datenspeicher 38 verbunden, um die ermittelte Kennlinie 30 oder das ermittelte Kennfeld 32 im Datenspeicher 38 zu hinterlegen.

Es versteht sich, dass auch denkbar ist, dass der Datenspeicher 38 mit der Eingangsschnittstelle 12 kommuniziert und verbunden ist und die Analyseeinheit 14 dazu ausgebildet ist, eine Kennlinie 30 oder ein Kennfeld 32 aus dem Datenspeicher 38 auszulesen und basierend auf der Kennlinie 30 oder dem Kennfeld 32 einen Zustand des Verbundbauteils zu bestimmen.

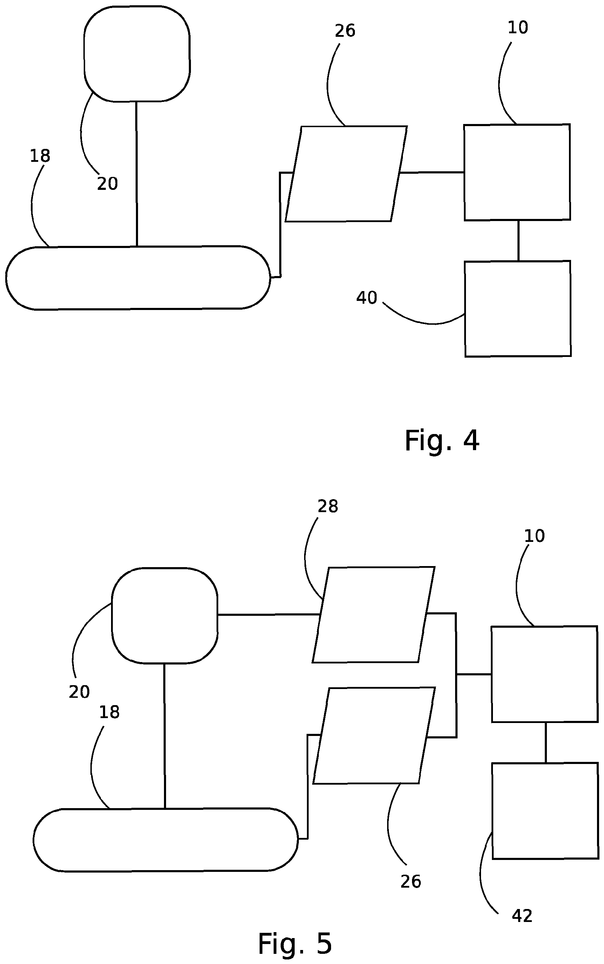

In Figur 4 ist schematisch ein Flussdiagramm zum Bestimmen von Belastungen in einem Verbundbauteil dargestellt.

Auf das Verbundbauteil 18 wirkt eine mechanische Anregung 20, insbesondere eine externe Belastung, wobei im Messgerät eine entstandene elektrische Ladungsverschiebung, elektrische Spannung und/oder Mikrofonie verstärkt und durch die Vorrichtung 10 empfangen und analysiert wird. Vorzugsweise kann mittels der Ausgangsschnittstelle 16 der Vorrichtung eine mechanische Belastung 40 des Verbundbauteils 18 ausgegeben werden.

In Figur 5 ist ein Flussdiagramm für eine Prognose einer Restlebensdauer eines Verbundbauteils 18 schematisch dargestellt.

Hierbei findet eine mechanische Anregung 20 in Form von externen Effekten wie Bewegungen und/oder Vibrationen statt, die auf das Verbundbauteil 18 einwirken und zusätzlich von einem Sensor 28 erfasst werden. Es versteht sich, dass der Sensor 28 effektbezogen gewählt wird und vorzugsweise einen Beschleunigungssensor umfassen kann. Die in dem Verbundbauteil 18 entstandene elektrische Ladungsverschiebung, elektrische Spannung und/oder Mikrofonie wird, wie bereits bezüglich Figur 4 beschrieben, in einem Messgerät verstärkt, wobei die Vorrichtung 10 sowohl Sensordaten des Sensors 28 als auch Messdaten des Messgeräts empfängt und in einer Datenverarbeitung verarbeitet. Insbesondere kann in der Analyseeinheit der Vorrichtung 10 eine Korrelation von externen Effekten, also der mechanischen Anregung 20, und der elektrischen Ladungsmessung, also dem Signal des Messgeräts bzw. Messverstärkers, erfolgen. Hierdurch kann eine Restlebensdauer 42 prognostiziert werden.

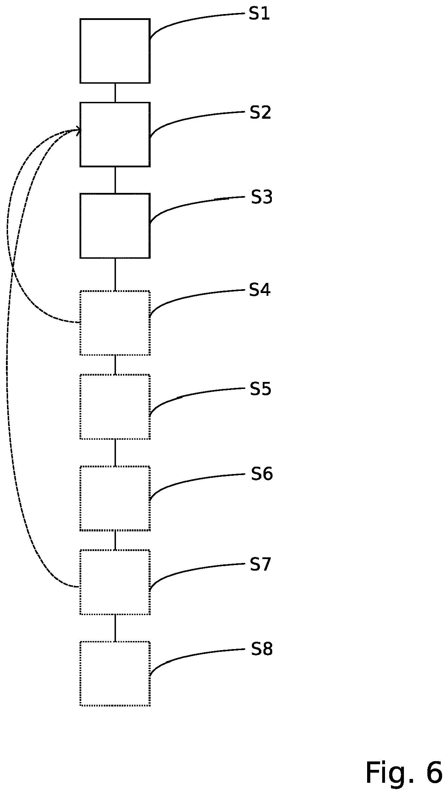

In Figur 6 sind schematisch die Schritte eines erfindungsgemäßen Verfahrens dargestellt.

In einem ersten Schritt S1 erfolgt ein Empfangen von Messdaten mit Informationen zu einem Zustand des Verbundbauteils.

In einem zweiten Schritt S2 wird ein Zustand des Verbundbauteils basierend auf den Messdaten ermittelt.

Schließlich erfolgt in einem dritten Schritt S3 ein Übermitteln des ermittelten Zustands des Verbundbauteils.

Die Messdaten umfassen eine elektrische Ladungstrennung und/oder eine elektrische Spannung, besonders bevorzugt eine Mikrofonie, die durch eine mechanische Anregung des Verbundbauteils erzeugbar ist bzw. erzeugt wurde.

Das Ermitteln des Zustands des Verbundbauteils umfasst ein Bestimmen des Zustands des Verbundbauteils anhand der elektrischen Ladungstrennung, elektrischen Spannung und/oder vorzugsweise Mikrofonie.

In einem optionalen vierten Schritt S4 erfolgt ein Empfangen von Sensordaten mit Informationen zu einer mechanischen Anregung des Verbundbauteils. Hierbei kann das Ermitteln eines Zustands des Verbundbauteils, also der zweite Schritt S2, ein Bestimmen des Zustands des Verbundbauteils basierend auf den Sensordaten und den Messdaten umfassen. In der Figur 6 ist das durch einen gestrichelten Pfeil dargestellt.

In einem weiteren oder alternativen optionalen fünften Schritt S5 kann ein Einwirken auf das Verbundbauteil mit einer vordefinierten Kraft erfolgen, um eine vordefinierte mechanische Anregung des Verbundbauteils zu erzeugen.

Dem optionalen fünften Schritt S5 folgt ein optionaler sechster Schritt S6, der ein Quantifizieren einer durch die vordefinierte mechanische Anregung des Verbundbauteils erzeugten Mikrofonie umfasst.

In einem optionalen siebten Schritt S7 erfolgt ein Bestimmen einer verbundbauteil-spezifischen Kennlinie und/oder eines verbundbauteil-spezifischen Kennfelds.

In einem besonders bevorzugten optionalen achten Schritt S8 erfolgt ein Übermitteln der verbundbauteil-spezifischen Kennlinie und/oder des verbundbauteil-spezifischen Kennfelds an einen Datenspeicher. Es versteht sich, dass das bestimmte Kennfeld oder die bestimmte Kennlinie auch im zweiten Schritt S2, dem Ermitteln eines Zustands des Verbundbauteils, berücksichtigt werden kann. Insbesondere kann bekanntes Wissen aus einer Datenbank beim Ermitteln des Zustands mit eingebracht werden. Es versteht sich, dass maschinelles Lernen Basis der Datenanalyse sein kann.

Folglich kann bevorzugt der zweite Schritt S2, Ermitteln eines Zustands des Verbundbauteils, ein Bestimmen einer einwirkenden mechanischen Belastung, vorzugsweise basierend auf einer verbundbauteil-spezifischen Kennlinie und/oder eines verbundbauteil-spezifischen Kennfelds, umfassen.

In einer weiteren bevorzugten Ausführungsform kann der zweite Schritt S2, das Ermitteln eines Zustands des Verbundbauteils, ein Bestimmen eines Verschlei-ßes, einer Fehlstelle in einem Material, einer Belastungshistorie und/oder einer Alterung eines Materials des Verbundbauteils basierend auf einer beliebigen mechanischen Anregung, vorzugsweise basierend auf einer verbundbauteil-spezifischen Kennlinie und/oder eines verbundbauteil-spezifischen Kennfelds, umfassen.

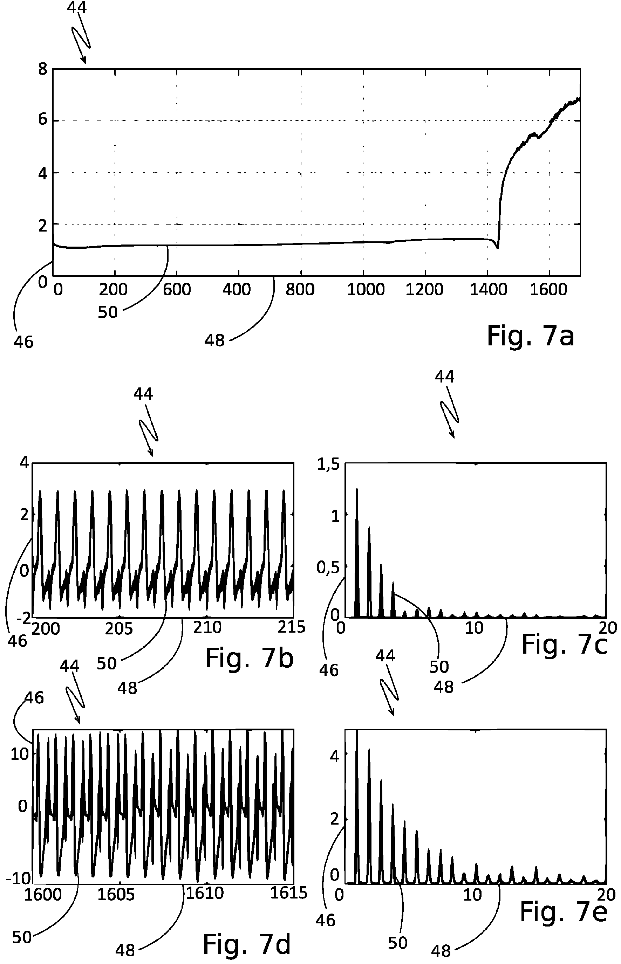

In Figur 7a ist ein Effektivwert einer elektrischen Ladungsverschiebung bei einem Biegeversuch über die Anzahl der Zyklen aufgetragen.

Bei diesem Biegeversuch wurde ein Koaxialkabel, insbesondere ein Koaxialkabei des Typs CLF200, gemäß der Biegeprüfung nach DIN EN50396 untersucht.

Das Koaxialkabel weist als Innenleiter einen starren Kupferleiter mit 1 mm$^2$ Querschnitt auf, der durch ein Dielektrikum in Form von Polyethylen (PE) von einer Schirmung isoliert ist. Die Schirmung umfasst eine Aluminiumfolie und ein Kupfergeflecht. Dieses Koaxialkabel weist einen Mantel aus PVC auf.

Bei dem Biegeversuch wird das Kabel mit einem Biegeradius von 50 mm, bei einem Winkel von ±90° und mit 60 Wiederholungen pro Minute belastet. Eine Brucherkennung erfolgt mittels einer Stromüberwachung, vorzugsweise mit 0,1 A, wobei die Innen- und Außenleiter in Reihe geschaltet sind.

Figur 7a zeigt ein Schaubild 44 des Effektivwerts der elektrischen Ladungsverschiebung über einer Anzahl von Zyklen bzw. Wiederholungen.

Auf der y-Achse ist der Effektivwert, beispielsweise der quadratische Mittelwert, RMS, der elektrischen Ladungsverschiebung in Wurzel aus Pico-Coulomb-Quadrat $\sqrt{pC^2}$ aufgetragen. Auf der x-Achse sind die Wiederholungen aufgetragen.

Die y-Achse 46 reicht in dem in Figur 7a gezeigten Schaubild 44 von 0 bis 8. Die x-Achse 48 reicht im in Figur 7a gezeigten Schaubild von 0 bis über 1600, wobei ein x-Achsenstrich jeweils 200 Zyklen markiert.

Ein Verlauf 50 des Effektivwerts der elektrischen Ladung weist im Bereich zwischen 0 und ca. 1.400 Wiederholungen einen etwa flachen Verlauf mit einem Wert für die effektive elektrische Ladungsverschiebung von ca. 1 Wurzel Pico Coulomb-Quadrat $\sqrt{pC^2}$ auf. Danach fällt der Verlauf 50 leicht ab und steigt stark an auf einen Wert von über 6 Wurzel Pico Coulomb-Quadrat $\sqrt{pC^2}$ bei über 1.600 Wiederholungen.

In Figur 7b ist ein Schaubild 44 eines zeitlichen Verlaufs der Wiederholungen 200 bis 215 gezeigt, wobei die y-Achse 46 einen Bereich von -2 bis 4 Pico-Coulomb pC umfasst.

In Figur 7c ist der in Figur 7b zeitlich dargestellte Verlauf im Frequenzbereich dargestellt, wobei die x-Achse 48 einen Bereich von 0 bis 20 Hz umfasst und die y-Achse 46 einen Bereich von 0 bis 1,5 Betrag Pico-Coulomb umfasst.

In den Figuren 7d und 7e ist in analoger Weise zu den Figuren 7b und 7c ein Verlauf im Zeitbereich und im Frequenzbereich dargestellt.

Im Unterschied zu dem in Figur 7b dargestellten Schaubild 44 reicht die y-Achse 46 in Figur 7d von -10 bis über 10 Pico-Coulomb *pC*, wobei die Wiederholungen 1600 bis 1615 dargestellt sind.

In Figur 7e reicht die y-Achse 46 von 0 bis über 4 Betrag Pico-Coloumb |*pC*|.

Beim Vergleich der Figuren 7b, 7c mit den Figuren 7d, 7e ist zu erkennen, dass die elektrische Ladungsverschiebung eine höhere Amplitude aufweist. Ferner ist zu erkennen, dass im Frequenzbereich die elektrische Ladungsverschiebung mehr Komponenten im höherfrequenzigen Bereich, insbesondere zwischen 10 und 20 Hz, aufweist. Zudem sind auch Frequenzanteile größer 5 Hz stärker ausgeprägt.

Die Erfindung wurde anhand der Zeichnungen und der Beschreibung umfassend beschrieben und erklärt. Die Beschreibung und Erklärung sind als Beispiel und nicht einschränkend zu verstehen. Die Erfindung ist nicht auf die offenbarten Ausführungsformen beschränkt. Andere Ausführungsformen oder Variationen ergeben sich für den Fachmann bei der Verwendung der vorliegenden Erfindung sowie bei einer genauen Analyse der Zeichnungen, der Offenbarung und der nachfolgenden Patentansprüche.

In den Patentansprüchen schließen die Wörter "umfassen" und "mit" nicht das Vorhandensein weiterer Elemente oder Schritte aus. Der undefinierte Artikel "ein" oder "eine" schließt nicht das Vorhandensein einer Mehrzahl aus. Ein einzelnes Element oder eine einzelne Einheit kann die Funktionen mehrerer der in den Patentansprüchen genannten Einheiten ausführen. Ein Element, eine Einheit, eine Vorrichtung und ein System können teilweise oder vollständig in Hard- und/oder in Software umgesetzt sein. Die bloße Nennung einiger Maßnahmen in mehreren verschiedenen abhängigen Patentansprüchen ist nicht dahingehend zu verstehen, dass eine Kombination dieser Maßnahmen nicht ebenfalls vorteilhaft verwendet werden kann. Ein Computerprogramm kann auf einem nichtflüchtigen Datenträger gespeichert/vertrieben werden, beispielsweise auf einem optischen Speicher oder auf einem Halbleiterlaufwerk (SSD). Ein Computerprogramm kann zusammen mit Hardware und/oder als Teil einer Hardware vertrieben werden, beispielsweise mittels des Internets oder mittels drahtgebundener oder drahtloser Kommunikationssysteme. Bezugszeichen in den Patentansprüchen sind nicht einschränkend zu verstehen.

Bezugszeichen

| 10 | Vorrichtung |
| 12 | Eingangsschnittstelle |
| 14 | Analyseeinheit |
| 16 | Ausgangsschnittstelle |
| 18 | Verbundbauteil |
| 20 | mechanische Anregung |
| 22 | Kabel |
| 24 | System |
| 26 | Ladungsverstärker |
| 28 | Sensor |
| 30 | Kennlinie |
| 32 | Kennfeld |
| 34 | Schaubild |
| 36 | Anregeeinheit |
| 38 | Datenspeicher |
| 40 | Belastung |
| 42 | Restlebensdauer |
| 44 | Schaubild |
| 46 | y-Achse |
| 48 | x-Achse |
| 50 | Verlauf |
| S1-S3 | Verfahrensschritte |
| S4-S8 | optionale Verfahrensschritte |

**Patentansprüche**

1. Vorrichtung (10) zum Bestimmen eines Zustands eines Verbundbauteils (18), umfassend:

eine Eingangsschnittstelle (12) zum Empfangen von Messdaten mit Informationen zu einem Zustand des Verbundbauteils (18);

eine Analyseeinheit (14) zum Ermitteln eines Zustands des Verbundbauteils (18) basierend auf den Messdaten; und
eine Ausgangsschnittstelle (16) zum Übermitteln des ermittelten Zustands; wobei
die Messdaten eine elektrische Ladungsverschiebung und/oder elektrische Spannung umfassen, die durch eine mechanische Anregung (20) des Verbundbauteils (18) durch das Verbundbauteil erzeugbar ist; und
die Analyseeinheit (14) dazu ausgebildet und eingerichtet ist, anhand der elektrischen Ladungstrennung und/oder elektrischen Spannung den Zustand des Verbundbauteils (18) zu bestimmen.

2. Vorrichtung (10) nach dem vorstehenden Anspruch, mit einem Messgerät, insbesondere in Form eines Ladungsverstärkers oder Spannungsverstärkers (26), zum Erhöhen eines Messsignals, insbesondere einer Spannungsamplitude der elektrischen Spannung.

3. Vorrichtung (10) nach einem der vorstehenden Ansprüche, wobei die Eingangsschnittstelle (12) zum Empfangen von Sensordaten mit Informationen zu einer mechanischen Anregung (20) des Verbundbauteils (18) ausgebildet ist; und
die Analyseeinheit (14) dazu ausgebildet und eingerichtet ist, basierend auf den Sensordaten den Zustand des Verbundbauteils (18) zu bestimmen.

4. Vorrichtung (10) nach einem der vorstehenden Ansprüche, wobei die Analyseeinheit (14) dazu ausgebildet und eingerichtet ist, eine durch eine mechanische Anregung (20) des Verbundbauteils (18) erzeugte Mikrofonie zu quantifizieren, und basierend auf der quantifizierten Mikrofonie einen Zustand, vorzugsweise umfassend einen Verschleiß, Fehlstellen in einem Material, eine Belastungshistorie und/oder eine Alterung eines Materials, des Verbundbauteils (18) zu bestimmen.

5. Vorrichtung (10) nach einem der vorstehenden Ansprüche, wobei die Analyseeinheit (14) dazu ausgebildet und eingerichtet ist, eine durch eine vordefinierte mechanische Anregung (20) des Verbundbauteils (18) erzeugte Mikrofonie zu quantifizieren, und basierend auf der quantifizierten Mikrofonie eine verbundbauteil-spezifische Kennlinie (30) und/oder ein verbundbauteil-spezifisches Kennfeld (32) zu bestimmen; und
vorzugsweise die Ausgangsschnittstelle (16) dazu ausgebildet und eingerichtet ist, die verbundbauteil-spezifische Kennlinie (30) und/oder das verbundbauteil-spezifische Kennfeld (32) an einen Datenspeicher (38) zu übermitteln.

**6.** System (24) zum Bestimmen eines Zustands eines Verbundbauteils (18), mit:

> einer Vorrichtung (10) nach einem der vorstehenden Ansprüche; und
> einer Anregeeinheit (36) zum Einwirken auf das Verbundbauteil (18) und Erzeugen einer vordefinierten mechanischen Anregung (20) des Verbundbauteils (18).

**7.** System (24) nach dem vorstehenden Anspruch, mit:

> einem Sensor (28) zum Erfassen einer mechanischen Anregung (20) des Verbundbauteils (18) und Erzeugen von Sensordaten mit Informationen zu der mechanischen Anregung des Verbundbauteils (18); wobei
> die Vorrichtung (10) dazu ausgebildet und eingerichtet ist, basierend auf den Sensordaten den Zustand des Verbundbauteils (18) zu bestimmen.

**8.** Verfahren zum Bestimmen eines Zustands eines Verbundbauteils (18) umfassend die Schritte:

> Empfangen (S1) von Messdaten mit Informationen zu einem Zustand des Verbundbauteils (18);
> Ermitteln (S2) eines Zustands des Verbundbauteils (18) basierend auf den Messdaten; und
> Übermitteln (S3) des ermittelten Zustands; wobei
> die Messdaten eine elektrische Ladungstrennung und/oder elektrische Spannung umfassen, die durch eine mechanische Anregung (20) des Verbundbauteils (18) durch das Verbundbauteil (18) erzeugt wird; und
> das Ermitteln (S2) eines Zustands des Verbundbauteils (18) ein Bestimmen des Zustands des Verbundbauteils (18) anhand der elektrischen Ladungstrennung und/oder elektrischen Spannung umfasst.

**9.** Verfahren nach dem vorstehenden Anspruch, mit dem Schritt:

> Empfangen (S4) von Sensordaten mit Informationen zu einer mechanischen Anregung (20) des Verbundbauteils (18); wobei
> das Ermitteln (S2) eines Zustands des Verbundbauteils (18) ein Bestimmen des Zustands des Verbundbauteils (18) basierend auf den Sensordaten umfasst.

**10.** Verfahren nach Anspruch 8 oder 9, mit den Schritten:

> Einwirken (S5) auf das Verbundbauteil (18) mit einer vordefinierten Kraft, um eine vordefinierte mechanische Anregung (20) des Verbundbauteils (18) zu erzeugen;
> Quantifizieren (S6) einer durch die vordefinierte mechanische Anregung (20) des Verbundbauteils (18) erzeugten Mikrofonie;
> Bestimmen (S7) einer verbundbauteil-spezifischen Kennlinie (30) und/oder eines verbundbauteil-spezifischen Kennfelds (32); und
> vorzugsweise Übermitteln (S8) der verbundbauteil-spezifischen Kennlinie (30) und/oder des verbundbauteil-spezifischen Kennfelds (32) an einen Datenspeicher (38).

**11.** Verfahren nach einem der Ansprüche 8 bis 10, wobei der Schritt Ermitteln (S2) eines Zustands des Verbundbauteils (18) ein Bestimmen einer einwirkenden mechanischen Belastung (40), vorzugsweise basierend auf einer verbundbauteil-spezifischen Kennlinie (30) und/oder eines verbundbauteil-spezifisches Kennfelds (32), umfasst.

**12.** Verfahren nach einem der Ansprüche 8 bis 11, wobei der Schritt Ermitteln (S2) eines Zustands des Verbundbauteils (18) ein Bestimmen eines Verschleißes, einer Fehlstelle in einem Material, einer Belastungshistorie und/oder einer Alterung eines Materials des Verbundbauteils (18), basierend auf einer beliebigen mechanischen Anregung (20), vorzugsweise basierend auf einer verbundbauteil-spezifischen Kennlinie (30) und/oder eines oder eines verbundbauteil-spezifischen Kennfelds (32), umfasst.

Fig. 1

Fig. 2

Fig. 3

Fig. 4

Fig. 5

Fig. 6

Fig. 7a

Fig. 7b

Fig. 7c

Fig. 7d

Fig. 7e

**Europäisches Patentamt / European Patent Office / Office européen des brevets**

# EUROPÄISCHER RECHERCHENBERICHT

**Nummer der Anmeldung**

EP 23 18 6617

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2019 206234 A1 (ZAHNRADFABRIK FRIEDRICHSHAFEN [DE]) 5. November 2020 (2020-11-05) * Zusammenfassung * * Absätze [0010], [0039], [0040] – [0042] * * Abbildung 1 * ----- | 1-12 | INV. G01M5/00 G01M7/08 |
| A | US 2019/025157 A1 (HEBRARD YOANN [FR]) 24. Januar 2019 (2019-01-24) * Absatz [0032] * ----- | 3 | |
| A | MUND B: "KABEL-MIKROFONIE- EXAKT GEMESSEN", ELEKTRONIK, W E K A FACHZEITSCHRIFTEN-VERLAG GMBH, DE, Bd. 45, Nr. 19, 17. September 1996 (1996-09-17), Seiten 80-84, XP000633385, ISSN: 0013-5658 * Zusammenfassung * ----- | 4,5 | |
| A | US 2012/151989 A1 (KNOX JOHN GRAEME [US] ET AL) 21. Juni 2012 (2012-06-21) * Absatz [0023] * * Abbildung 1 * ----- | 6,10 | RECHERCHIERTE SACHGEBIETE (IPC) G01M |
| A | EP 0 351 430 A1 (MITSUI SHIPBUILDING ENG [JP]) 24. Januar 1990 (1990-01-24) * Abbildung 1 * ----- | 6,10 | |

Der vorliegende Recherchenbericht wurde für alle Patentansprüche erstellt

| Recherchenort | Abschlußdatum der Recherche | Prüfer |
|---|---|---|
| Den Haag | 24. November 2023 | Grewe, Clemens F. |

EPO FORM 1503 03.82 (P04C03)

**EP 4 312 013 A1**

## ANHANG ZUM EUROPÄISCHEN RECHERCHENBERICHT
## ÜBER DIE EUROPÄISCHE PATENTANMELDUNG NR.

**EP 23 18 6617**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

**24-11-2023**

| Im Recherchenbericht angeführtes Patentdokument | Datum der Veröffentlichung | Mitglied(er) der Patentfamilie | Datum der Veröffentlichung |
|---|---|---|---|
| DE 102019206234 A1 | 05-11-2020 | KEINE | |
| US 2019025157 A1 | 24-01-2019 | CN 109269689 A | 25-01-2019 |
| | | DE 102017212283 A1 | 24-01-2019 |
| | | US 2019025157 A1 | 24-01-2019 |
| US 2012151989 A1 | 21-06-2012 | BR 112013012063 A2 | 16-08-2016 |
| | | CN 103250040 A | 14-08-2013 |
| | | EP 2652477 A1 | 23-10-2013 |
| | | JP 2014510259 A | 24-04-2014 |
| | | KR 20130114204 A | 16-10-2013 |
| | | RU 2013132956 A | 27-01-2015 |
| | | US 2012151989 A1 | 21-06-2012 |
| | | WO 2012082477 A1 | 21-06-2012 |
| EP 0351430 A1 | 24-01-1990 | KEINE | |

EPO FORM P0461

Für nähere Einzelheiten zu diesem Anhang : siehe Amtsblatt des Europäischen Patentamts, Nr.12/82

16